# EUROPEAN PATENT SPECIFICATION

(11) **EP 3 326 517 B1**
(45) Date of publication and mention of the grant of the patent: **16.09.2020**
(21) Application number: 16200175.4
(22) Date of filing: 23.11.2016
(51) Int. Cl.: A61B 5/00, A61B 5/0402, A61B 5/046, G16H 50/20

(54) **METHOD AND SYSTEM FOR IDENTIFYING POTENTIAL ATRIAL FLUTTER AREAS FOR MEDICAL DECISION SUPPORT**
VERFAHREN UND SYSTEM ZUM IDENTIFIZIEREN VON POTENZIELLEN VORHOFFLATTERBEREICHEN ZUR MEDIZINISCHEN ENTSCHEIDUNGSUNTERSTÜTZUNG
PROCÉDÉ ET SYSTÈME D'IDENTIFICATION DU FLUTTER AURICULAIRE POTENTIEL POUR AIDER À LA DÉCISION MÉDICALE

(43) Date of publication of application: 30.05.2018
(73) Proprietor: Karlsruher Institut für Technologie, 76131 Karlsruhe (DE); Städtisches Klinikum Karlsruhe gGmbH, 76133 Karlsruhe (DE)
(72) Inventor: OESTERLEIN, Tobias, 75038 Obererdingen (DE); DÖSSEL, Olaf, 76646 Bruchsal (DE); SCHMITT, Claus, 69121 Heidelberg (DE); LURK, Armin, 76187 Karlsruhe (DE)
(74) Representative: Bittner, Peter

(56) References cited:
- WO-A1-03/094721
- WO-A1-2015/120064
- WO-A1-2015/120095
- P. PASCALE ET AL: "Pattern and Timing of the Coronary Sinus Activation to Guide Rapid Diagnosis of Atrial Tachycardia After Atrial Fibrillation Ablation", CIRCULATION. ARRHYTHMIA AND ELECTROPHYSIOLOGY, vol. 6, no. 3, 1 June 2013 (2013-06-01), pages 481-490, XP055373841, United States ISSN: 1941-3149, DOI: 10.1161/CIRCEP.113.000182

## Description

### Technical Field

The present invention generally relates to electronic data processing, and more particularly, relates to methods, computer program products and systems for identifying potential atrial flutter maintaining areas based on clinical sensor data.

### Background

Atrial flutter can frequently be observed as either index arrhythmia or as organized tachycardia following the ablation of atrial fibrillation (AFib). In the latter case, atrial flutter (AFlut) often is resistant to pharmacological treatment and cardioversion, but as well seen as intermediate step before normal sinus rhythm (SR) is achieved. In both cases, however, numerous studies have already demonstrated consistent results with respect to the underlying mechanisms and their anatomical locations. This understanding and the resulting treatment decisions have led to success rates in the order of 80 to 100% when catheter ablation is applied to terminate AFlut. The understanding of the patient-specific variant, however, is the central prerequisite of successful ablation. Details regarding the medical details can be found in the following references: "A classification of atrial flutter and regular atrial tachycardia according to electrophysiological mechanisms and anatomical bases; a Statement from a Joint Expert Group from The Working Group of Arrhythmias of the European Society of Cardiology and the North American Society of Pacing and Electrophysiology (by Saoudi, N.; Cosio, F.; Waldo, A.; Chen, S. A.; lesaka, Y.; Lesh, M.; Saksena, S.; Salerno, J.; Schoels, W.; European Heart Journal; 2001; 22; 1162-1182)"; "Atrial tachycardia after ablation of persistent atrial fibrillation: identification of the critical isthmus with a combination of multielectrode activation mapping and targeted entrainment mapping; (by Patel, A. M.; d'Avila, A.; Neuzil, P.; Kim, S. J.; Mela, T.; Singh, J. P.; Ruskin, J. N.; Reddy, V. Y.; 2008; Circulation. Arrhythmia and Electrophysiology; 1: 14-22)"; and "Characterization, Mapping and Ablation of Complex Atrial Tachycardia: Initial Experience with a Novel Method of Ultra High-Density 3D Mapping; (by Schaeffer, B.; Hoffmann, B. A.; Meyer, C.; Akbulak, R. O.; Moser, J.; Jularic, M.; Eickholt, C.; Nuhrich, J. M.; Kuklik, P.; Willems, S.; 2016; Journal of Cardiovascular Electrophysiology; 27: 1139-1150)".

Currently, this diagnostic process is primarily based on the experience of the physician. Although comprehensive electrogram data can be acquired from the complete atrium using an electro-anatomical mapping system (EAMS) with multi-polar mapping catheters, only two automatic analysis techniques are known. First, the maximum bipolar voltage of electrograms (EGMs) is evaluated within one cycle. Regions which exhibit voltage values below a certain threshold are considered scar, potentially being part of an anatomical obstacle. Typical threshold values for atrial myocardium are in the range of 0.5mV for zones of low voltage and 0.05mV for dense scar. Second, the local activation time (LAT) is annotated in each signal. After all signals are synchronized, the LAT reflects the global excitation pattern. This static image allows to gain a first impression of the course of atrial activation.

However, a number of additional features are assessed by physicians in the diagnostic process and typically evaluated manually by visual inspection. Considering the analysis of individual signals, these comprise the presence of double potentials, and evaluation of fractionation and duration of activity in each signal. From a multichannel point of view, additional features can be determined, like the mid-diastolic activity, area-based cycle length coverage and the critical isthmus. In addition to the references above by Patel et al. and Schaeffler et al., further medical details regarding the additional features are disclosed in the references: "A deductive mapping strategy for atrial tachycardia following atrial fibrillation ablation: importance of localized reentry; (by Jais, P.; Matsuo, S.; Knecht, S.; Weerasooriya, R.; Hocini, M.; Sacher, F.; Wright, M.; Nault, I.; Lellouche, N.; Klein, G.; Clementy, J.; Haissaguerre, M.; 2009; Journal of Cardiovascular Electrophysiology; 20; 480-491)", and "Treatment of macro-re-entrant atrial tachycardia based on electroanatomic mapping: identification and ablation of the mid-diastolic isthmus; (by De Ponti, R.; Verlato, R.; Bertaglia, E.; Del Greco, M.; Fusco, A.; Bottoni, N.; Drago, F.; Sciarra, L.; Ometto, R.; Mantovan, R.; Salerno-Uriarte, J. A.; 2007; Europace : European Pacing, Arrhythmias, and Cardiac Electrophysiology : Journal of the Working Groups on Cardiac Pacing, Arrhythmias, and Cardiac Cellular Electrophysiology of the European Society of Cardiology; 9; 449-457)".

The PCT application WO2015120064 discloses a method to analyze electrical data for a plurality of signals of interest at different spatial sites within identified zones of a patient's anatomical structure to determine intracardiac signal characteristics for the plurality of sites within each respective zone. The method generates an output that integrates at least one mechanism of distinct arrhythmogenic electrical activity for one or more zones with intracardiac signal characteristics for the plurality of sites within each respective zone. A percentage continuous activation calculator is disclosed which uses annotation of local activation time related to the common standard approach of computation by calculating activation time. An active interval is identified around each local activation detection which is converted into the percentage of continuous activation as the ratio of active portion to passive portion. This approach follows clinical practice to identify arrhythmogenic regions by their tendency to show long fractionated signals, making them targets for catheter ablation as described in "A new approach for catheter ablation of atrial fibrillation: mapping of the electrophysiologic substrate", Journal of the American College of Cardiology, 43/11, pp. 2044-2053, 2004, 10.1016/j.jacc.2003.12.054". An intracardiac characteristic aggregator is suggested to average the computed characteristics for sites within a given zone, which is in line with the identification of arrhythmogenic substrate containing prologed activity. But the identification of reentrant mechanisms is purely related to phase analysis. The flood of clinical data measured by corresponding sensors is overwhelming for any physician and does not appropriately support a physician in his/her effort in diagnosing atrial flutter types and locations for a patient.

### Summary

Therefore, there is a need to provide a diagnostic decision support solution with a corresponding method to assess these parameters automatically in order to support a medically trained human (e.g., a physician) in understanding the flutter circuit for improved diagnostic decision making as a basis for successful ablation.

This technical problem is solved by the features of a computer system, a computer-implemented method and a computer program product as disclosed in the independent claims.

In one embodiment, the computer system is a decision support system for supporting diagnostic analysis of atrial flutter types. The system includes an interface component to receive an electrical reference signal generated by a reference sensor. Typically, a coronary sinus catheter sensor (CSCS) is used as a reference sensor. However, a reference sensor may also be placed at a location different from the coronary sinus in the patient's atria. In one embodiment, an ECG sensor may also be used as a reference sensor. The reference signal provides a time reference for electrical activity of one or more atria of a patient wherein the time interval between two subsequent electrical activities is referred to as basic cycle length (BCL). Time reference, as used herein, means that any delay times of signals measured by the further sensors at particular locations are measured in relation to the signal measured by the reference sensor.

In one embodiment, the reference sensor may provide a position reference. Position reference, as used herein, means that the position of each further sensor is known relative to the position of the reference sensor. Often, the CSCS is used as the position reference. Typically, the location of the sensors in the heart are determined via magnet coils mounted underneath the patient. Once the position of the reference sensor is determined the positions of other sensors are known relative to the position of the reference sensor. If the patient moves, the relative positions can be determined because the movement of the reference sensor is detected. It is also possible to determine the movement of the patient by other means. For example, the movement may be detected by a camera system or by ultrasonic sensors. The detected movement can then be used to re-compute the sensor locations by compensating the movement accordingly. In these implementations, the position reference function of the reference sensor is optional. Further, the interface receives a plurality of electrical signals generated by one or more further sensors wherein the plurality of electrical signals relate to a plurality of locations in the one or more atria. The plurality of locations is different from the location of the reference sensor (CSCS). In other words, the electrical signals received by the one or more further sensors illustrate how the excitation wave propagates through the atrium of a patient before and after passing the coronary sinus as detected by the reference sensor. The signals may be received by the interface directly from the sensors, thus enabling a live monitoring of the atria activity or it may be retrieved from a database which stores historic measurement data of the sensors.

The system further has a signal analyzer component to determine, for each signal of at least a subset of the received signals originating from locations within a selected area of the one or more atria, an active interval when electrical activity occurs, and a resting interval when no electrical activity occurs. Electrical activity hereby is a consequence of the depolarization of cardiac tissue close to the sensor. No electrical activity occurs when neighboring tissue is in rest and does not depolarize. Thus the electrical activity reflects changes of the trans-membrane voltage of adjacent myocardial cells. For example, the selected area may correspond to an atrium. The selection may be predefined (e.g., the right atrium or the left atrium) or it may be received by the system via an appropriate user interface from a user of the system. For example, a physician may use a pointing mechanism (e.g., by using a mouse, a touch screen, or the like) to indicate selected areas of interest in a graphical visualization of the patient's heart. In other words, the physician may select both atria, only one atrium, or just a part of an atrium as area of interest for the later diagnostic analysis.

Further, the signal analyzer computes, based on the determined intervals (active intervals and resting intervals), a subset cycle length coverage as the ratio of duration of activity and the basic cycle length. Thereby, the duration of activity includes the active intervals for the entire subset of signals. In other words, the duration of activity corresponds to one or more intervals which result from superposing all active intervals detected by the further sensors located within the selected area.

A visualizer component of the system indicates to the medically trained human the selected area (e.g., right or left atrium) as an area including a potential focal source if the subset cycle length coverage is smaller than a predefined ratio. If the subset cycle length coverage is equal of above the predefined ratio, the selected area is indicated as an area including a potential re-entry. The indication may be implemented through visualization parameters setting a predefined color or pattern for the visualization of the identified atrial flutter type. "Focal source" and "re-entry" are the major atrial flutter types as they are known by the medically trained user of the system. For example, when a potential focal source is determined in the selected area, the selected area can be visualized to the medically trained person in a first color or using a first pattern associated with the atrial flutter type "focal source". When a potential re-entry is determined in the selected area, a second color or second pattern may be used which is associated with the atrial flutter type "re-entry".

A first indicator for the nature of the underlying tachycardia mechanism is given by the amount of cycle length which can be annotated when all recorded signals are assessed in conjunction. Reentry is typically suspected as mechanism for atrial flutter if activation times for at least 95% of the BCL (i.e., predefined ratio: 95%) can be successfully mapped. For each moment of the covered time, accordingly, an EGM can be found somewhere in the atrium which exhibits activity and whose local activity time can be annotated. Inversely, no activity can be detected for less than 15% of the cycle length. Given a sufficiently high-density of intracardiac measurements, the atrium is suspected to be in rest during this inactive time, excluding the presence of a re-entrant source and endorsing a truly focal source. The coverage of macro re-entry activation was shown to be about 95.9±4.3% (range 90% to 100 %) in clinical mapping studies (cf., the above cited De Ponti reference). This is one important indicator that can be assessed when all recorded electrograms are analyzed jointly.

The buffer of 15% is frequently applied since some endocardial aspects of the flutter cycle may not be reachable during mapping, or the flutter may propagate on the epicardial aspect of the atrium. Thus the excitation wave cannot be observed at these locations and no LAT can be annotated. In addition, also highly fractionated potentials are assigned just one LAT value, despite the fact that they may cover significant parts of the cycle length. Studies have shown prolonged activation at the critical isthmus lasting mean durations of 200±80 ms, with individual potentials having duration of 360 ms as illustrated in "Flutter localized to the anterior left atrium after catheter ablation of atrial fibrillation (by Jais, P.; Sanders, P.; Hsu, L.-F.; Hocini, M.; Sacher, F.; Takahashi, Y.; Rotter, M.; Rostock, T.; Bordachar, P.; Reuter, S.; Laborderie, J.; Clementy, J.; Haissaguerre, M.; 2006; Journal of Cardiovascular Electrophysiology; 17; 279-285)". This demonstrated a situation in which the annotation of LAT will not lead to optimal results. Instead, an activity based approach is suggested in the following.

Although the annotation of LAT may not lead to optimal results, in one embodiment, a predefined LAT may be used to define a surrogate for the activity. Therefore, a time window between 10 milliseconds and 300 milliseconds (advantageously between 20ms or 30ms) around the LAT may be defined as active interval. This may be done independently of assessing the signal morphology, and thus not reflect the true activity. However, it may be used as an initial interval length which may already lead to results reflecting the general flutter type. For example, in this embodiment, the active interval (i.e. the predefined window) may be centered around a particular point in time where the respective signal shows activity. The particular time point can be the maximum or the minimum of the signal amplitude during a basic cycle length, or it may be the maximum of the absolute value of the time derivative of the signal. Typically, the activity of the signal swings first to a maximum value of the signal amplitude and then to a minimum value of the signal amplitude before turning back to a value close to zero (resting interval). Between the time points with maximum and minimum values of the signal amplitude the absolute value of the time derivative reaches its maximum. The predefined window may be centered around any one of these time points.

In one embodiment, the system can provide additional information regarding the type of atrial flutter re-entry. This flutter type includes two sub-types: micro-re-entry and macro-re-entry. Both atrial flutter types require different treatment of the patient. Therefore, it is advantageous if the medically trained person is provided with the additional information about the subtype of a potential re-entry. In this embodiment, once a potential re-entry is detected in the selected area, the signal analyzer component further can select a plurality of sub-areas within the originally selected area wherein the sub-areas have a size which is smaller than the size of the selected area, and wherein each sub-area has an overlapping area with at least one further sub-area. For example, the sub-areas may have a circular shape or another geometric shape (e.g., square, rectangle, triangle, etc.) which is appropriate to sub-divide the selected area into evenly spread sub-areas with overlapping areas between two neighboring sub-areas.

The signal analyzer can then compute for each sub-area the respective sub-area cycle length coverage. This computation is performed in an analogous manner to the computation of the sub-set cycle length coverage. However, instead of using the subset of signals originating from locations within the selected area for determining the active intervals and resting intervals only signals originating from locations within the respective sub-area are used for the computation. In other words, for the computation of a particular sub-area cycle length coverage the corresponding ratio of duration of activity and the basic cycle length takes into account the active intervals for the signals originating from a location within the particular sub-area.

Because of the higher spatial granularity of the sub-area cycle length coverages, there may be two or more neighboring sub-areas with different sub-area cycle length coverage values leading to a conflict situation of the respective overlapping area. To solve this problem with this embodiment, for each overlapping area with conflicting sub-area cycle length coverages from at least two overlapping sub-areas, one or more predefined conflict resolution rules are applied to determine a respective overlapping area cycle length coverage based on the conflicting sub-area cycle length coverages. For example, any one of the following conflict resolution rules may be used by the signal analyzer: assigning the conflicting sub-area cycle length coverage with the highest value to the respective overlapping area cycle length coverage; assigning the average value of the conflicting sub-area cycle length coverages to the respective overlapping area cycle length coverage; and assigning a weighted average value of the conflicting sub-area cycle length coverages to the respective overlapping area cycle length coverage.

In the case of the atrial flutter type "focal source" the duration of activity is approximately the same in all sub-areas. However, the origin of the focal source can be determined to be in the sub-area where the activity occurs first in time. In other words, the sub-area showing the earliest activity can be proposed to the physician as a potential target for later ablation.

Further, in this embodiment, the visualizer component can indicate sub-areas and overlapping areas with respective cycle length coverages equal or above an atrial flutter type threshold as areas including a potential micro re-entry, and further indicate sub-areas and overlapping areas with respective cycle length coverages below the atrial flutter type threshold as areas not including a potential micro re-entry. If no sub-area or overlapping area indicates the presence of a micro re-entry, a conclusion for the presence of a macro re-entry can be drawn. The indication at the level of sub-areas may be implemented through visualization parameters setting a predefined color or pattern associates with the respective atrial flutter type in a similar way as for the selected areas.

The spatial resolution regarding the identification of potential locations comprising an atrial flutter source depends on the size and shape of the sub-areas. In one embodiment, the sub-areas have a circular shape and the overlapping areas between two overlapping circles account for 1/5 to 1/3 of the sub-area diameter. In an alternative embodiment, the sub-areas have a circular shape with a diameter between 2 cm and 4 cm, and the circle centers of two neighboring sub-areas have a distance between 0,5 cm and 1,5 cm. In another embodiment, the sub-areas have a square shape with a diagonal between 2 cm and 4 cm, and the square centers of two neighboring sub-areas have a distance between 0,5 cm and 1,5 cm. In an alternative embodiment, the sub-areas have a square shape and the overlapping areas between two overlapping circles account for 1/5 to 1/3 of the sub-area diagonal. A person skilled in the art can use other geometric forms for the sub-areas and achieve a comparable coverage of the selected area with a comparable resolution by applying similar overlapping ratios as disclosed for circle and square shaped sub-areas.

In one embodiment, a computer-implemented method is provided for supporting diagnostic analysis of atrial flutter types. The method may be executed by the previously disclosed computer system. The method includes: receiving an electrical reference signal generated by a reference sensor, the reference signal providing a position reference and a time reference for electrical activity of one or more atria of a patient wherein the time interval between two subsequent electrical activities is referred to as basic cycle length; receiving a plurality of electrical signals generated by one or more further sensors wherein the plurality of electrical signals relate to a plurality of locations in the one or more atria, the plurality of locations being different from the location of the reference sensor; determining, for each signal of at least a subset of the received signals originating from locations within a selected area of the one or more atria (e.g., one atrium of the patient), an active interval when electrical activity occurs, and a resting interval when no electrical activity occurs; based on the determined intervals, computing a subset cycle length coverage as the ratio of duration of activity and the basic cycle length wherein the duration of activity includes the active intervals for the entire subset of signals; and if the subset cycle length coverage is smaller than a predefined ratio then indicating the selected area as an area including a potential focal source, else indicating the selected area as an area including a potential re-entry.

Computing a subset cycle length coverage includes applying a Boolean OR operator to the determined activity and resting intervals of the respective signals so that any activity interval of any signal contributes to the duration of activity, and no activity in all signals contributes to the duration of resting.

In one embodiment, the method further includes: if the selected area includes a potential re-entry, selecting a plurality of sub-areas within the originally selected area wherein the sub-areas have a size which is smaller than the size of the selected area, and wherein each sub-area has an overlapping area with at least one further sub-area; computing for each sub-area the respective sub-area cycle length coverage; for each overlapping area with conflicting sub-area cycle length coverages from at least two overlapping sub-areas, applying one or more predefined conflict resolution rules to determine a respective overlapping area cycle length coverage based on the conflicting sub-area cycle length coverages; indicating sub-areas and overlapping areas with respective cycle length coverages equal to or above an atrial flutter type threshold as areas including a potential micro re-entry. Optionally, the method may include indicating sub-areas and overlapping areas with respective cycle length coverages below the atrial flutter type threshold as areas including a potential macro re-entry. The presence of a macro re-entry can be concluded If no sub-area or overlapping area indicates the presence of a micro re-entry. As previously disclosed, the indication of the various atrial flutter types can be controlled by corresponding visualization parameter associated with the respective atrial flutter types. The visualization parameters may represent predefined different colors, patterns, animations, image depth in 3D visualizations, etc. for the various flutter types. When the system visualizes a particular atrial flutter type, the associated visualization parameter is used to render the respective area (selected area or sub-areas) in a graphical representation of the patient's atria.

For example, a predefined conflict resolution rule can include assigning the conflicting sub-area cycle length coverage with the highest value to the respective overlapping area cycle length coverage. In this example, the visualization parameter for the respective overlapping area equals the visualization parameter of the corresponding sub-area with the highest value.

For example, a predefined conflict resolution rule can include assigning the average value of the conflicting sub-area cycle length coverages to the respective overlapping area cycle length coverage. In this example, a visualization parameter for the overlapping area is computed based on visualization parameters of the sub-areas having conflicting cycle length coverages. For example, if the visualization parameter of a first sub-area indicates a red visualization and the visualization parameter of a second sub-area indicates a blue visualization, the visualization parameter of the overlapping area may result in a violet visualization. The average value may be computed based on RAL values of the respective colors. Other number values describing colors may be used instead. In case of using pattern based visualization parameters, in one example the first sub-area may have the visualization parameter horizontal hatch and the second sub-area may have a vertical hatch pattern. The average may be computed as the overlay of both hatch patterns resulting in a cross hatch. In the case of using animations (e.g., blinking of the respective area) the animation frequency may be the visualization parameter for averaging.

For example, a predefined conflict resolution rule can include assigning a weighted average value of the conflicting sub-area cycle length coverages to the respective overlapping area cycle length coverage. A person skilled in the art can adjust the conflict resolution rules for assigning average values by simply adding weighting factors which may overemphasize, for example, the visualization parameter of the sub-area having the sub-area cycle length coverage with the highest value.

In one embodiment, a computer program product is provided for supporting diagnostic analysis of atrial flutter types. The computer program product, when loaded into a memory of a computing device and executed by at least one processor of the computing device, can execute the steps of the computer-implemented method and implements the features of the disclosed decision support computer system.

Further aspects of the invention will be realized and attained by means of the elements and combinations particularly depicted in the appended claims. It is to be understood that both, the foregoing general description and the following detailed description are exemplary and explanatory only and are not restrictive of the invention as described.

### Brief Description of the Drawings

FIG. 1 is a simplified block diagram illustrating an embodiment of a decision support computer system for supporting diagnostic analysis of atrial flutter types;
FIG. 2A is a simplified flowchart of a computer-implemented method for supporting diagnostic analysis of atrial flutter types according to an embodiment of the invention;
FIG. 2B is a flow chart illustrating further embodiments of the computer-implemented method;
FIG. 3 illustrates activation intervals of a patient's atrium derived from sensor signals reflecting activation for particular locations of the atrium;
FIG. 4 illustrates the duration of activity and the duration of resting for the determination of cycle length coverage;
FIG. 5 is a simplified illustration of overlapping sub-areas on a graphic representation of an atrium according to one embodiment of the invention; and
FIG. 6 is a diagram that shows an example of a generic computer device and a generic mobile computer device which may be used with the techniques described herein.

### Detailed Description

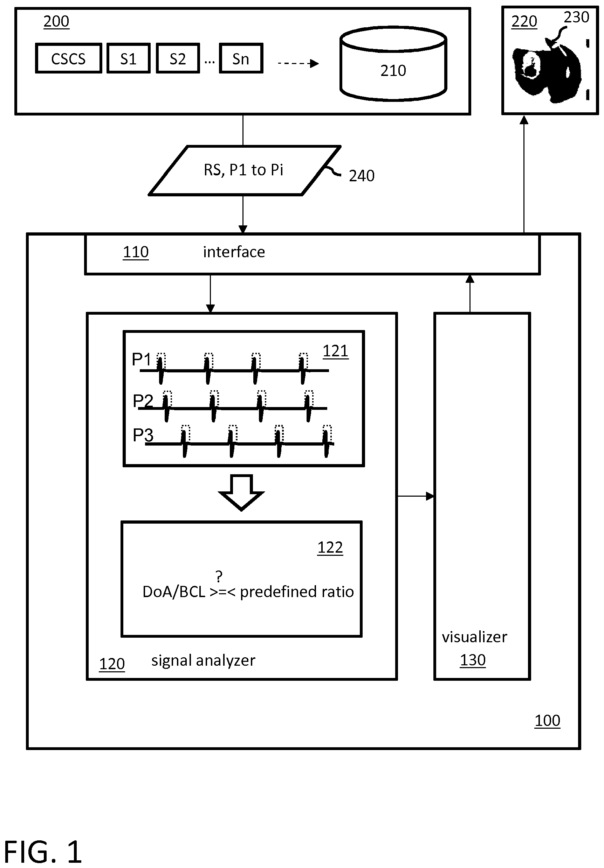

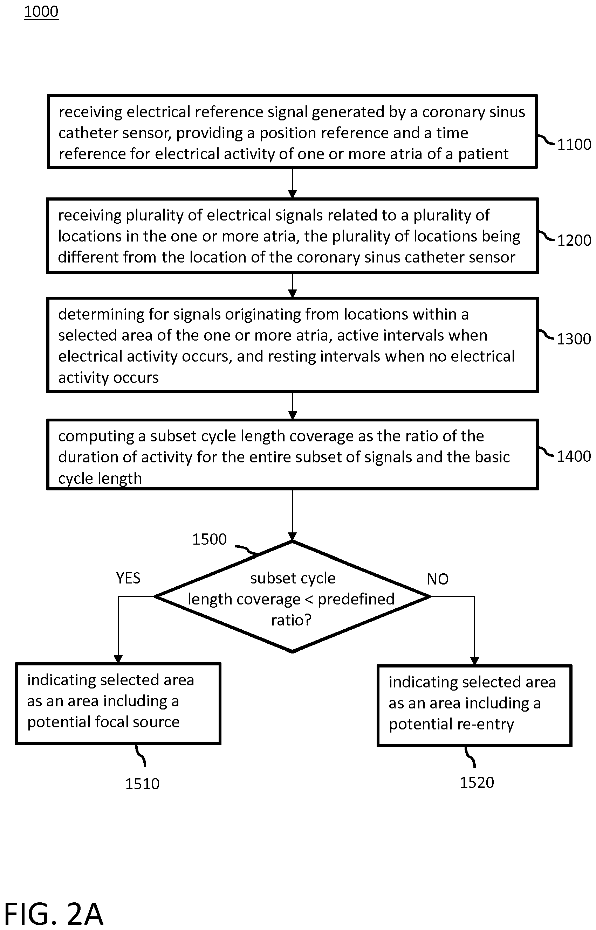

FIG. 1 is a simplified block diagram illustrating an embodiment of a decision support computer system 100 for supporting diagnostic analysis of atrial flutter types. FIG. 2A is a simplified flowchart of a computer-implemented method 1000 for supporting diagnostic analysis of atrial flutter types according to an embodiment of the invention. The functions of system 100 of FIG. 1 are discussed in the context of the method steps of method 1000 which are performed by the respective system components of system 100. Therefore, the following description refers to reference numbers of the FIGs. 1 and 2A.

The system 100 includes an interface component 110 configured to receive 1100, 1200 data 240 from one or more external data sources 200. The external data sources may be sensors CSCS, S1 to Sn providing real time data about the electric activation of a patient's atria or it may be a data storage device 210 which provides historic (previously recorded or simulated) sensor data about the electric activation of the patient's atria.

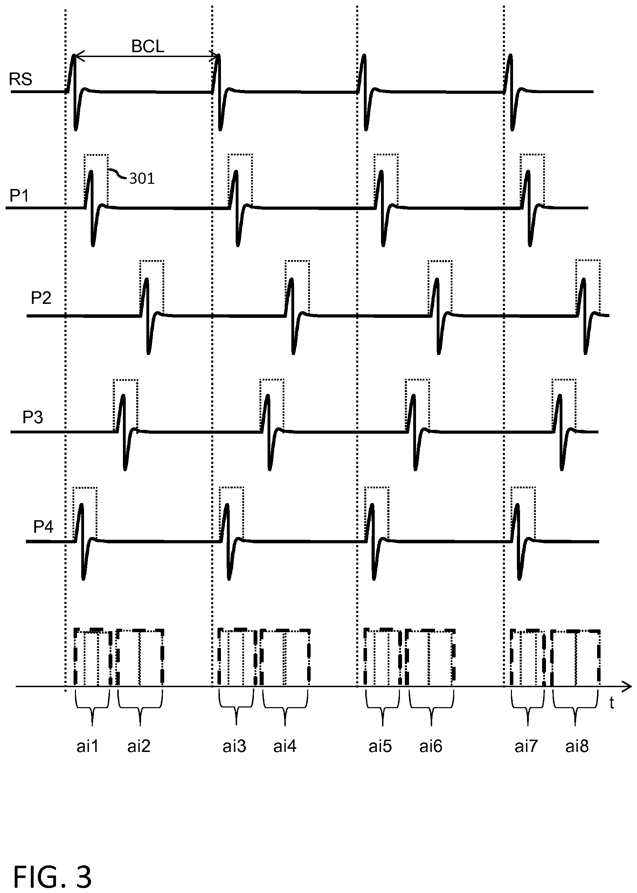

The received 1100 data 240 includes an electrical reference signal RS generated by a reference sensor. Typically, a coronary sinus catheter sensor CSCS is used to measure the electric activity in a patient's atria at the location of the coronary sinus. This reference signal RS provides a position reference and a time reference for electrical activity of one or more atria of the patient. Thereby, the time interval between two subsequent electrical activities is referred to as basic cycle length (BCL). More details are described in the context of FIG. 3.

The interface further receives 1200 a plurality of electrical signals P1 to Pi generated by one or more further sensors S1 to Sn wherein the plurality of electrical signals P1 to Pi relate to a plurality of locations in the one or more atria, the plurality of locations being different from the location of the reference sensor. Typically, such sensors are multi-polar mapping catheters which are widely used for electro-anatomical mapping systems EAMS. Examples of such sensors are described in detail in the references: "A multi-purpose spiral high-density mapping catheter: initial clinical experience in complex atrial arrhythmias; (by Jones, D. G.; McCready, J. W.; Kaba, R. A.; Ahsan, S. Y.; Lyne, J. C.; Wang, J.; Segal, O. R.; Markides, V.; Lambiase, P. D.; Wong, T.; Chow, A. W. C.; 2011; Journal of Interventional Cardiac Electrophysiology: an International Journal of Arrhythmias and Pacing; 31: 225-235)", and "Rapid high resolution electroanatomical mapping: evaluation of a new system in a canine atrial linear lesion model; (by Nakagawa, H.; Ikeda, A.; Sharma, T.; Lazzara, R.; Jackman, W. M.; 2012; Circulation. Arrhythmia and Electrophysiology; 5; 417-424)".

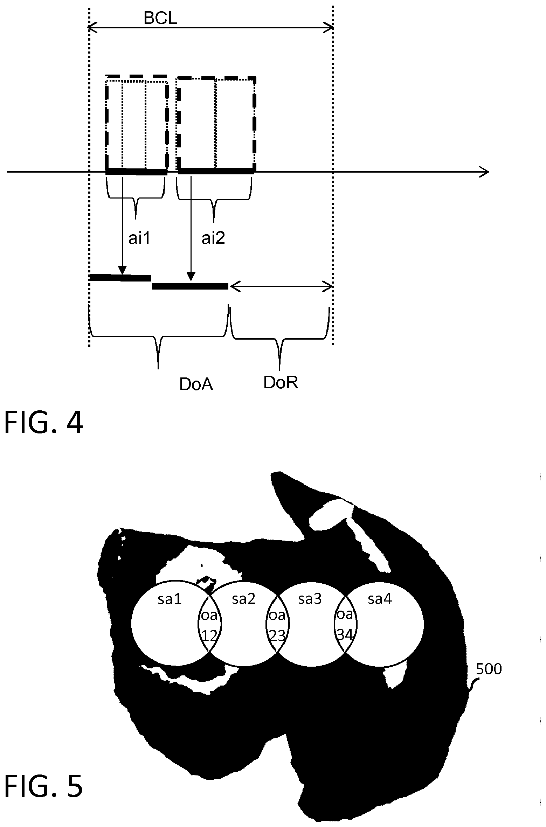

The system 100 further has a signal analyzer component 120. The signal analyzer 120 determines, for each signal P1, P2, P3 of at least a subset 121 of the received signals P1 to Pi if an electrical activity occurs at the location of the respective sensor. Thereby, the subset 121 includes such signals P1, P2, P3 which originate from locations within a selected area of the one or more atria. Typically, the selected area is one atrium (of the patient's heart) which is subject to the diagnostic analysis. The selected area may also relate to a portion of an atrium. The selection of the selected area may be received from the medically trained person performing the diagnosis or it may be predefined in the system 100. For example, in a first analysis the right atrium (or a portion) may be selected and in a second analysis the left atrium (or a portion) may be selected. Based on the activity intervals of the various signals in the subset 121, for each signal in the subset 121, the signal analyzer determines 1300 an active interval when electrical activity occurs, and further determines 1300 a resting interval when no electrical activity occurs. For example, the detection of activity may be performed by assessing the amplitude of the measured signal in that a window is shifted over the signal with the maximum and minimum amplitude being computed and the current window being identified as activity during an active interval if the difference between maximum and minimum values exceeds a certain threshold. In an alternative implementation, the detection of activity may be performed by assessing the instantaneous energy of the measured signal, in which a threshold can be used to distinguish active and inactive parts of the signal. The durations of the electrical activity of the signals P1, P2, P3 in subset 121 are illustrated by the rectangular shapes which are superposed to the signal curves. More details are discussed in the context of FIGs. 3 and 4.

Based on the determined intervals, a comparator component 122 of the signal analyzer 120 computes 1400 a subset cycle length coverage as the ratio of duration of activity (DoA) and the basic cycle length BCL wherein the duration of activity includes the active intervals for the entire subset 121 of signals. In other words, the DoA corresponds to the total active time of the atrium in the selected area which is monitored by the sensors providing the signals of the subset 121. The comparator 122 compares 1500 the determined subset cycle length coverage to a predefined ratio. According to studies (e.g., the study illustrated in the above cited reference "A multi-purpose spiral high-density mapping catheter ..." by Jones et al.) it is known that the predefined ratio which can be used to distinguish the selected area with regards to the atrial flutter types focal source and re-entry is approximately 95% (the exact number may vary according to different studies). If the selected area is an area which includes a potential focal source then the subset cycle length coverage determined from the signals of the subset 121 is smaller than said predefined ratio. If the subset cycle length coverage is equal or above said predefined ratio, the selected area is an area including a potential re-entry.

By using the above disclosed signal analysis the system 100 can compute an indicator for the clinical state (atrium flutter type) of the patient's atrium which is analyzed. This clinical state is then conveyed by the system 100 to the medically trained person to support the diagnosis and take a decision for the appropriate treatment of the patient. For this purpose, the system includes a visualizer component 130 which indicates 1510 the selected area as an area including a potential focal source if the subset cycle length coverage is smaller than a predefined ratio, and else indicates 1520 the selected area as an area including a potential re-entry. As discussed above, visual parameters, such as color, patterns or animations may be used to render a graphic representation 230 of the patient's active atria tissue on a display device 220 (e.g., communicatively coupled with the system 100 via interface 110) in such a way that the medically trained user of the system is provided with clear information about the potential clinical state (focal source vs. re-entry) of the patient's atria which facilitates the diagnostic analysis by the user. Any appropriate display technology (e.g., monitor, touch screen, etc.) may be used for the visualization of the atrial flutter types.

In one embodiment, an additional filter function may be used by the signal analyzer to discard signals from the subset which may be tampered with pulses from the patient's heart's ventricles. In this embodiment, a surface electrogram ECG can also be recorded simultaneously to the intracardiac electrical signals P1 to Pi, and can then be analyzed to detect ventricular depolarizations (QRS complexes). Intracardiac signals may be compromised by far fields originating from ventricular depolarizations. This may lead to electrical activity in the intracardiac signal causing deviations from baseline and thus potentially being misinterpreted as activity originating from an atrial source. Thus the activity of the atrium should be assessed while no QRS is present. The filter function may now discard or ignore any signals of the subset during such intervals where QRS is present.

FIG. 3 illustrates activation intervals of a patient's atrium derived from sensor signals reflecting activation for particular locations of the atrium. In the example, the reference signal RS is shown at the top providing the location and time reference for the signals P1 to P4 which are taken by sensors at locations different from the location of the reference sensor. In this example, one further signal P4 is part of the subset of signals received from sensors monitoring the selected area. That is, the signals P1 to P4 are all taken into account when performing the atrial flutter type analysis as disclosed in FIGs. 1 and 2A. The reference signal RS illustrates the periodic activity observed at the coronary sinus. The time between two voltage amplitude peaks is defined as the basic cycle length BCL. Besides maxima in the voltage, also other characteristic morphological markers of the electrical signal, such as for example, minima, strongest gradient, and so forth can be used to assign the reference time. As the reference signal corresponds to an electrical signal which is generated by a human organ and not by a clock signal of a technical system, there may be small variability in the BCL length over time. This has been discussed in the Saoudi reference cited above. Continuously monitoring the CS catheter reference signal allows to compensate for such small variability in BCL by scaling the duration of the resulting active and inactive periods. For example, every 300 milliseconds a variance of +/- 15 milliseconds may occur (modulation of BCL caused by breathing). By comparing the activity intervals with the corresponding actual BCL, the computed subset cycle length coverage is automatically normalized with regards to the actual BCL. The actual BCL can be manually defined by the medically trained person or can be based on a median value of all measured actual BCL or it may be based on a mean value of all measured actual BCL.

The signal analyzer determines for each signal P1 to P4 the activity intervals for each cycle. In the example of FIG. 3 a cycle is indicated as the time between two of the horizontal dotted lines. However, it is not relevant where a cycle starts and ends as long as the cycle length corresponds to BCL. The activity intervals of the respective signals are illustrated by the rectangular single overlays. For simplicity, only the rectangular overlay 301 is shown with a reference number. The resting interval of a cycle is defined as the difference between BCL and the length of the respective active interval of the signal.

For computing the subset cycle length coverage, the analyzer component firstly determines the duration of activity for the active intervals for the entire subset of signals P1 to P4. A Boolean OR operator is applied to the rectangular shapes (e.g., 301) representing the lengths of the determined intervals of respective signals so that any activity interval of the signals of the subset contributes to the duration of activity. To determine the overall duration of resting (across the signals of the subset). With regards to the duration of resting, only periods with no activity in all of the signals can contribute when a Boolean OR operator is applied. The bottom line of FIG. 3 illustrates the result of the OR operator applied to the signals P1 to P4. In each cycle two activity intervals are detected which are illustrated by the dashed frames. In the first cycle, the Boolean OR application results in the activity intervals ai1, ai2. In the second cycle the result is ai3, ai4, and so forth.

FIG. 4 illustrates the duration of activity DoA and the duration of resting DoR for the determination of cycle length coverage. The determined activity intervals ai1, ai2 of the first cycle are added to provide the overall duration of activity DoA. By computing the ratio between the duration of DoA and BCL the subset cycle length coverage is computed for the selected area which is monitored by the subset of signals P1 to P4. It is to be noted that in a real world diagnosis scenario the number of signals in a subset can easily exceed 1000 in cases where a database is providing historic sensor data. In real time monitoring scenarios the number of simultaneously received signals is limited by the number of sensors which can be operated simultaneously in the patient's heart. It is to be noted that historic sensor data, as used herein, may have been collected very recently (e.g., a few seconds or minutes before being processed by the signal analyzer). That is, although not representing real-time data, historic sensor data may also reflect the current medical state of the patient's atria.

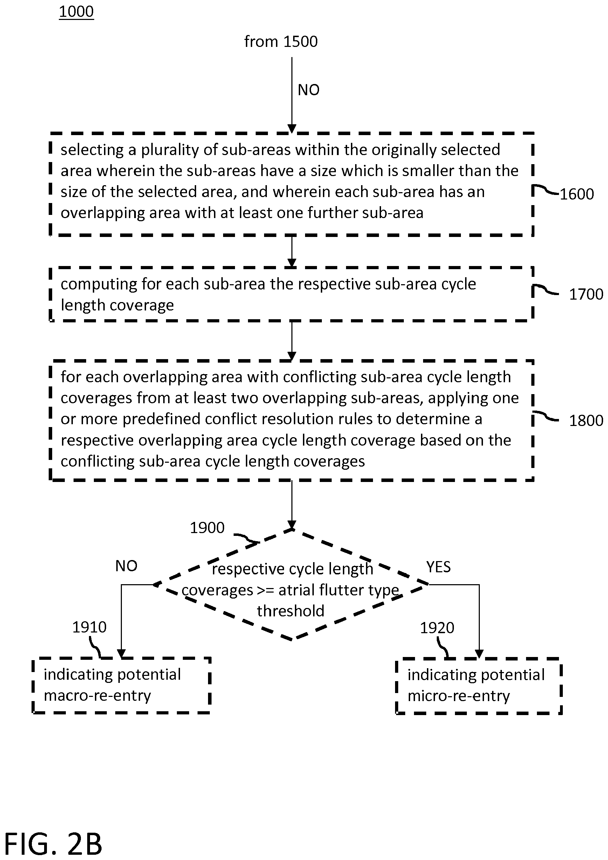

FIG. 5 is a simplified example of overlapping sub-areas sa1 to sa4 on a graphic representation 500 (corresponding to the selected area) of an atrium according to an embodiment of the invention. FIG. 5 will be described in the context of the simplified flowchart of FIG. 2B which illustrates further steps of the computer-implemented method performed by the decision support system to provide more detailed information about the atrial flutter types to the medically trained user for supporting the diagnosis process. Therefore, the following description refers to reference numbers of FIG. 5 and FIG. 2B. The further steps of the method 1000 are illustrated by dashed frames indicating that these steps are to be perceived as optional when performing method 1000.

Upon detection of a potential re-entry in the selected area (i.e., If the subset cycle length coverage is determined 1500 equal or above the predefined ratio), the signal analyzer component further selects 1600 a plurality of sub-areas sa1 to sa4 within the originally selected area 500. The sub-areas are automatically selected by the signal analyzer component according to predefined selection rules so that each sub-area has a size which is smaller than the size of the selected area 500, and each sub-area has an overlapping area oa12 to oa34 with at least one further sub-area. The predefined selection rules may include a predefined shape for sub-areas and predefined overlapping parameters. For example, in one embodiment, the selection rules may define the sub-areas as having a circular shape with a diameter between 2 and 4 cm. The sub-area dimensions relate to the respective heart portion covered by a particular sub-area (and not to the dimensions of the visualization of the heart which may be magnified or demagnified). The overlapping parameters may define that the overlapping areas between two overlapping circles account for 1/5 to 1/3 of the sub-area diameter. In an alternative embodiment, the overlapping parameters may define circle centers of two neighboring sub-areas having a distance between 0,5 cm and 1,5 cm. In another embodiment, the shape parameters may define a square shape with a diagonal between 2 cm and 4 cm, and the overlapping parameters may define square centers of two neighboring sub-areas having a distance between 0,5 cm and 1,5 cm. In an alternative embodiment, the sub-areas have a square shape and the overlapping areas between two overlapping circles account for 1/5 to 1/3 of the sub-area diagonal. A person skilled in the art can use other geometric forms for the sub-areas and achieve a comparable coverage of the selected area with a comparable resolution by applying similar overlapping ratios as disclosed for circle and square shaped sub-areas. Further, the selection rules may define particular portions of the selected area as areas of interest with regards the analysis of atrial flutter types. Such areas of interest may be received from the medically trained user or they may be predefined in the system. The signal analyzer can then distribute the sub-areas according the corresponding selection rules across the entire selected area or across one or more particular portions (areas of interest) of the selected area.

In the example of FIG. 5, for the reason of simplicity, only one row of overlapping sub-areas sa1 to sa2 with circle shapes is shown. The skilled person, however, will understand that further rows may exist above and below the shown row which have further overlapping areas with the sub-areas sa1 to sa4. The selection rules in this example can define such further rows of overlapping sub-areas in such a way that the circle centers would be positioned on a vertical line defined by the intersection points of two neighboring sub-areas. In this example, each overlapping area has overlap portions where three sub-areas are overlapping. In another implementation, the selection rules can define such further rows of overlapping sub-areas in such a way that the circle centers would be positioned on a vertical line intersecting with the circle center of the respective above or below sub-area. In this implementation the overlapping area may relate to two or four sub-areas depending on the size parameters of the respective sub-areas. A person skilled in the art can define other appropriate selection rules to achieve sufficient coverage of the entire selected area or selected areas of interest with appropriate overlapping sub-areas.

The signal analyzer can then compute 1700 the respective sub-area cycle length coverage for each sub-area sa1 to sa4. The computation may be performed by the same algorithm which is used for the previous computation of the subset cycle length coverage for the selected areas. However, only the signals originating from sensors at locations covered by the respective sub-area are used for the computation of the respective sub-area cycle length coverage.

It may occur that different sub-area cycle length coverage values are computed for neighboring sub-areas. In this case, there is a conflict with regards to the cycle length coverage to be assigned to the overlapping areas oa12, oa23, oa34 which is defined as the intersection of the overlapping sub-areas sa1-sa2, sa2-sa3, and sa3-sa4, respectively. To resolve this conflict, for each overlapping area with conflicting sub-area cycle length coverages from at least two overlapping sub-areas, the signal analyzer applies 1800 one or more predefined conflict resolution rules to determine a respective overlapping area cycle length coverage based on the conflicting sub-area cycle length coverages. Different approaches for conflict resolution can be defined by different conflict resolution rules. For example, the signal analyzer may assign the conflicting sub-area cycle length coverage with the highest value to the respective overlapping area cycle length coverage as a high sensitivity to high cycle length coverage may be desired to identify potential micro-reentry atrial flutter mechanisms. In another example, it may assign the average value of the conflicting sub-area cycle length coverage values to the respective overlapping area cycle length coverage. In a further example, it may assign a weighted average value of the conflicting sub-area cycle length coverage values to the respective overlapping area cycle length coverage.

Once the sub-area cycle length coverage values and overlapping area cycle length coverage values are computed, the signal analyzer compares 1900 the computed values to an atrial flutter type threshold which may be different from the predefined ratio used by the first comparison 1500 (cf. FIG. 2A) for distinguishing between the atrial flutter types focal source and re-entry. However, the atrial flutter type threshold used by the second comparison 1900 may also be equal or similar to the predefined ratio. The atrial flutter type threshold is chosen as a threshold value which can be used to distinguish between the atrial flutter sub-types "micro-re-entry" and "macro-re-entry". Different medical studies propose different threshold values for this purpose. For example a threshold of 75% of BCL covered by the DoA within a sub-area of 2cm diameter is proposed in the previously cited work "A deductive mapping strategy for atrial tachycardia following atrial fibrillation ablation: importance of localized reentry" by Jais et al.. A cycle length coverage value below the atrial flutter type threshold is seen as an indicator for a potential "macro-re-entry". A cycle length coverage value equal to or above the atrial flutter type threshold is seen as an indicator for a potential "micro-re-entry".

The signal analyzer provides the result of the comparison 1900 regarding each sub-area sa1 to sa4 and each overlapping area oa12, oa23, oa34 to the visualizer component which can render a visualization of the sub-areas and overlapping areas accordingly. The rendering is performed in such a way that it indicates 1920 to the medically trained person a potential micro-re-entry if the respective cycle length coverage value is equal to or above the atrial flutter type threshold. Further, the rendering may indicate 1910 a potential macro-re-entry if the respective cycle length coverage value is below the atrial flutter type threshold (as described above). The indication is implemented via similar visualization parameters as disclosed already above for the rendering of the selected area. That is, different colors, patterns, animations, 3D-effects, etc. may be used to graphically distinguish areas with potential micro-re-entries from areas with potential macro-re-entries for the medically trained user to support the diagnostic decision making.

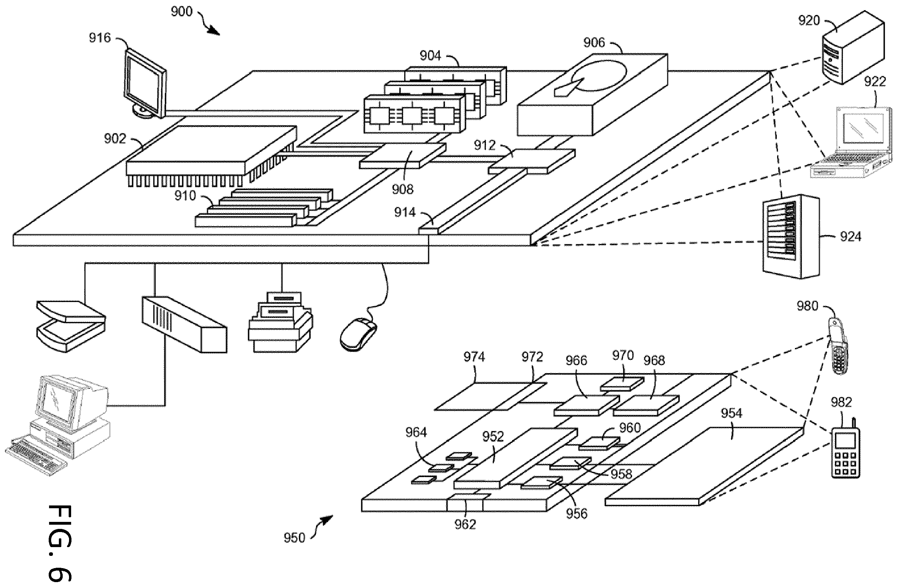

FIG. 6 is a diagram that shows an example of a generic computer device 900 and a generic mobile computer device 950, which may be used with the techniques described here. Computing device 900 is intended to represent various forms of digital computers, such as laptops, desktops, workstations, personal digital assistants, tablets, servers, blade servers, mainframes, and other appropriate computers. Generic computer device 900 may correspond to a computer system 100 as illustrated in FIG. 1. Computing device 950 is intended to represent various forms of mobile devices, such as personal digital assistants, cellular telephones, smart phones, and other similar computing devices. For example, computing device 950 may be used by a user as a front end to interact with the computer system 100. Computing device may, for example, include the display device 220 of FIG. 1. The components shown here, their connections and relationships, and their functions, are meant to be exemplary only, and are not meant to limit implementations of the inventions described and/or claimed in this document.

Computing device 900 includes a processor 902, memory 904, a storage device 906, a high-speed interface 908 connecting to memory 904 and high-speed expansion ports 910, and a low speed interface 912 connecting to low speed bus 914 and storage device 906. Each of the components 902, 904, 906, 908, 910, and 912, are interconnected using various busses, and may be mounted on a common motherboard or in other manners as appropriate. The processor 902 can process instructions for execution within the computing device 900, including instructions stored in the memory 904 or on the storage device 906 to display graphical information for a GUI on an external input/output device, such as display 916 coupled to high speed interface 908. In other implementations, multiple processing units and/or multiple buses may be used, as appropriate, along with multiple memories and types of memory. Also, multiple computing devices 900 may be connected, with each device providing portions of the necessary operations (e.g., as a server bank, a group of blade servers, or a processing device).

The memory 904 stores information within the computing device 900. In one implementation, the memory 904 is a volatile memory unit or units. In another implementation, the memory 904 is a non-volatile memory unit or units. The memory 904 may also be another form of computer-readable medium, such as a magnetic or optical disk.

The storage device 906 is capable of providing mass storage for the computing device 900. In one implementation, the storage device 906 may be or contain a computer-readable medium, such as a floppy disk device, a hard disk device, an optical disk device, or a tape device, a flash memory or other similar solid state memory device, or an array of devices, including devices in a storage area network or other configurations. A computer program product can be tangibly embodied in an information carrier. The computer program product may also contain instructions that, when executed, perform one or more methods, such as those described above. The information carrier is a computer- or machine-readable medium, such as the memory 904, the storage device 906, or memory on processor 902.

The high speed controller 908 manages bandwidth-intensive operations for the computing device 900, while the low speed controller 912 manages lower bandwidth-intensive operations. Such allocation of functions is exemplary only. In one implementation, the high-speed controller 908 is coupled to memory 904, display 916 (e.g., through a graphics processor or accelerator), and to high-speed expansion ports 910, which may accept various expansion cards (not shown). In the implementation, low-speed controller 912 is coupled to storage device 906 and low-speed expansion port 914. The low-speed expansion port, which may include various communication ports (e.g., USB, Bluetooth, Ethernet, wireless Ethernet) may be coupled to one or more input/output devices, such as a keyboard, a pointing device, a scanner, or a networking device such as a switch or router, e.g., through a network adapter.

The computing device 900 may be implemented in a number of different forms, as shown in the figure. For example, it may be implemented as a standard server 920, or multiple times in a group of such servers. It may also be implemented as part of a rack server system 924. In addition, it may be implemented in a personal computer such as a laptop computer 922. Alternatively, components from computing device 900 may be combined with other components in a mobile device (not shown), such as device 950. Each of such devices may contain one or more of computing device 900, 950, and an entire system may be made up of multiple computing devices 900, 950 communicating with each other.

Computing device 950 includes a processor 952, memory 964, an input/output device such as a display 954, a communication interface 966, and a transceiver 968, among other components. The device 950 may also be provided with a storage device, such as a microdrive or other device, to provide additional storage. Each of the components 950, 952, 964, 954, 966, and 968, are interconnected using various buses, and several of the components may be mounted on a common motherboard or in other manners as appropriate.

The processor 952 can execute instructions within the computing device 950, including instructions stored in the memory 964. The processor may be implemented as a chipset of chips that include separate and multiple analog and digital processing units. The processor may provide, for example, for coordination of the other components of the device 950, such as control of user interfaces, applications run by device 950, and wireless communication by device 950.

Processor 952 may communicate with a user through control interface 958 and display interface 956 coupled to a display 954. The display 954 may be, for example, a TFT LCD (Thin-Film-Transistor Liquid Crystal Display) or an OLED (Organic Light Emitting Diode) display, or other appropriate display technology. The display interface 956 may comprise appropriate circuitry for driving the display 954 to present graphical and other information to a user. The control interface 958 may receive commands from a user and convert them for submission to the processor 952. In addition, an external interface 962 may be provided in communication with processor 952, so as to enable near area communication of device 950 with other devices. External interface 962 may provide, for example, for wired communication in some implementations, or for wireless communication in other implementations, and multiple interfaces may also be used.

The memory 964 stores information within the computing device 950. The memory 964 can be implemented as one or more of a computer-readable medium or media, a volatile memory unit or units, or a non-volatile memory unit or units. Expansion memory 984 may also be provided and connected to device 950 through expansion interface 982, which may include, for example, a SIMM (Single In Line Memory Module) card interface. Such expansion memory 984 may provide extra storage space for device 950, or may also store applications or other information for device 950. Specifically, expansion memory 984 may include instructions to carry out or supplement the processes described above, and may include secure information also. Thus, for example, expansion memory 984 may act as a security module for device 950, and may be programmed with instructions that permit secure use of device 950. In addition, secure applications may be provided via the SIMM cards, along with additional information, such as placing the identifying information on the SIMM card in a non-hackable manner.

The memory may include, for example, flash memory and/or NVRAM memory, as discussed below. In one implementation, a computer program product is tangibly embodied in an information carrier. The computer program product contains instructions that, when executed, perform one or more methods, such as those described above. The information carrier is a computer- or machine-readable medium, such as the memory 964, expansion memory 984, or memory on processor 952, that may be received, for example, over transceiver 968 or external interface 962.

Device 950 may communicate wirelessly through communication interface 966, which may include digital signal processing circuitry where necessary. Communication interface 966 may provide for communications under various modes or protocols, such as GSM voice calls, SMS, EMS, or MMS messaging, CDMA, TDMA, PDC, WCDMA, CDMA2000, or GPRS, EDGE, UMTS, LTE, among others. Such communication may occur, for example, through radio-frequency transceiver 968. In addition, short-range communication may occur, such as using a Bluetooth, WiFi, or other such transceiver (not shown). In addition, GPS (Global Positioning System) receiver module 980 may provide additional navigation- and location-related wireless data to device 950, which may be used as appropriate by applications running on device 950.

Device 950 may also communicate audibly using audio codec 960, which may receive spoken information from a user and convert it to usable digital information. Audio codec 960 may likewise generate audible sound for a user, such as through a speaker, e.g., in a handset of device 950. Such sound may include sound from voice telephone calls, may include recorded sound (e.g., voice messages, music files, etc.) and may also include sound generated by applications operating on device 950.

The computing device 950 may be implemented in a number of different forms, as shown in the figure. For example, it may be implemented as a cellular telephone 980. It may also be implemented as part of a smart phone 982, personal digital assistant, or other similar mobile device.

Various implementations of the systems and techniques described here can be realized in digital electronic circuitry, integrated circuitry, specially designed ASICs (application specific integrated circuits), computer hardware, firmware, software, and/or combinations thereof. These various implementations can include implementation in one or more computer programs that are executable and/or interpretable on a programmable system including at least one programmable processor, which may be special or general purpose, coupled to receive data and instructions from, and to transmit data and instructions to, a storage system, at least one input device, and at least one output device.

These computer programs (also known as programs, software, software applications or code) include machine instructions for a programmable processor, and can be implemented in a high-level procedural and/or object-oriented programming language, and/or in assembly/machine language. As used herein, the terms "machine-readable medium" and "computer-readable medium" refer to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term "machine-readable signal" refers to any signal used to provide machine instructions and/or data to a programmable processor.

To provide for interaction with a user, the systems and techniques described here can be implemented on a computer having a display device (e.g., a CRT (cathode ray tube) or LCD (liquid crystal display) monitor) for displaying information to the user and a keyboard and a pointing device (e.g., a mouse or a trackball) by which the user can provide input to the computer. Other kinds of devices can be used to provide for interaction with a user as well; for example, feedback provided to the user can be any form of sensory feedback (e.g., visual feedback, auditory feedback, or tactile feedback); and input from the user can be received in any form, including acoustic, speech, or tactile input.

The systems and techniques described here can be implemented in a computing device that includes a backend component (e.g., as a data server), or that includes a middleware component (e.g., an application server), or that includes a front end component (e.g., a client computer having a graphical user interface or a Web browser through which a user can interact with an implementation of the systems and techniques described here), or any combination of such backend, middleware, or frontend components. The components of the system can be interconnected by any form or medium of digital data communication (e.g., a communication network). Examples of communication networks include a local area network ("LAN"), a wireless local area network ("WLAN"), a wide area network ("WAN"), and the Internet.

The computing device can include clients and servers. A client and server are generally remote from each other and typically interact through a communication network. The relationship of client and server arises by virtue of computer programs running on the respective computers and having a client-server relationship to each other.

A number of embodiments have been described. Nevertheless, it will be understood that various modifications may be made without departing from the scope of the invention.

In addition, the logic flows depicted in the figures do not require the particular order shown, or sequential order, to achieve desirable results. In addition, other steps may be provided, or steps may be eliminated, from the described flows, and other components may be added to, or removed from, the described systems. Accordingly, other embodiments are within the scope of the following claims.

## Claims

1. A decision support computer system (100) for supporting diagnostic analysis of atrial flutter types, comprising:
an interface component (110) configured to receive an electrical reference signal (RS) generated by a reference sensor (CSCS), the reference signal (RS) providing a time reference for electrical activity of one or more atria of a patient wherein the time interval between two subsequent electrical activities is referred to as basic cycle length (BCL), and further configured to receive a plurality of electrical signals (P1 to Pi) generated by one or more further sensors (S1 to Sn) wherein the plurality of electrical signals (P1 to Pi) relate to a plurality of locations in the one or more atria, the plurality of locations comprising locations different from the location of the reference sensor (CSCS);
a signal analyzer component (120) configured to determine, for each signal of at least a subset of the received signals originating from locations within a selected area (500) of the one or more atria, an active interval (ai1 to ai8) when electrical activity occurs, and a resting interval when no electrical activity occurs; and configured to compute, based on the determined intervals, a subset cycle length coverage as the ratio of duration of activity (DoA) and the basic cycle length (BCL) wherein the duration of activity includes the active intervals for the entire subset of signals; and further configured to apply a Boolean OR operator to the determined activity and resting intervals of respective signals so that any activity interval of any signal contributes to the duration of activity, and no activity in all signals contributes to the duration of resting; and
a visualizer component (130) configured to indicate the selected area as an area including a potential focal source if the subset cycle length coverage is smaller than a predefined ratio, and else to indicate the selected area as an area including a potential re-entry.

2. The system of claim 1, wherein the selected area corresponds to an atrium.

3. The system of any of previous claims, wherein the predefined defined ratio is between 75% and 100%.

4. The system of any of the previous claims, wherein, upon detection of a potential re-entry in the selected area, the signal analyzer component is further configured to:
select a plurality of sub-areas (sa 1 to sa4) within the originally selected area (500) wherein the sub-areas have a size which is smaller than the size of the selected area,
and wherein each sub-area has an overlapping area (oa12 to oa34) with at least one further sub-area;
compute for each sub-area (sa 1 to sa4) the respective sub-area cycle length coverage;
for each overlapping area with conflicting sub-area cycle length coverages from at least two overlapping sub-areas, to apply one or more predefined conflict resolution rules to determine a respective overlapping area cycle length coverage based on the conflicting sub-area cycle length coverages; and
the visualizer component (130) further configured to:
indicate sub-areas and overlapping areas with respective cycle length coverages equal to or above the atrial flutter type threshold as areas including a potential micro re-entry.

5. The system of claim 4, wherein the sub-areas have a circular shape and the overlapping areas between two overlapping circles account for 1/5 to 1/3 of the sub-area diameter, or wherein the sub-areas have a circular shape with a diameter between 2 cm and 4 cm, and wherein the circle centers of two neighboring sub-areas have a distance between 0,5 cm and 1,5 cm.

6. The system of claim 4 or 5, wherein the one or more predefined conflict resolution rules are selected from the group of rules including: assigning the conflicting sub-area cycle length coverage with the highest value to the respective overlapping area cycle length coverage; assigning the average value of the conflicting sub-area cycle length coverages to the respective overlapping area cycle length coverage; and assigning a weighted average value of the conflicting sub-area cycle length coverages to the respective overlapping area cycle length coverage.

7. A computer-implemented method (1000) for supporting diagnostic analysis of atrial flutter types, the method comprising:
receiving (1100) an electrical reference signal generated by a reference sensor, the reference signal providing a time reference for electrical activity of one or more atria of a patient wherein the time interval between two subsequent electrical activities is referred to as basic cycle length;
receiving (1200) a plurality of electrical signals generated by one or more further sensors wherein the plurality of electrical signals relate to a plurality of locations in the one or more atria, the plurality of locations comprising locations different from the location of the reference sensor;
determining (1300), for each signal of at least a subset of the received signals originating from locations within a selected area of the one or more atria, an active interval when electrical activity occurs, and a resting interval when no electrical activity occurs;
based on the determined intervals, computing (1400) a subset cycle length coverage as the ratio of duration of activity (DoA) and the basic cycle length (BCL) by applying a Boolean OR operator to the determined activity and resting intervals of the respective signals so that any activity interval of any signal contributes to the duration of activity, and no activity in all signals contributes to the duration of resting, wherein the duration of activity (DoA) includes the active intervals for the entire subset of signals;
if the subset cycle length coverage is smaller than a predefined ratio then indicating (1510) the selected area as an area including a potential focal source, else indicating (1520) the selected area as an area including a potential re-entry.

8. The method of any of claim 7, wherein the predefined defined ratio is between 75% and 100%.

9. The method of any of the claims 7 to 8, if the selected area includes a potential re-entry, the method further comprising:
selecting (1600) a plurality of sub-areas within the originally selected area wherein the sub-areas have a size which is smaller than the size of the selected area, and
wherein each sub-area has an overlapping area with at least one further sub-area;
computing (1700) for each sub-area the respective sub-area cycle length coverage;
for each overlapping area with conflicting sub-area cycle length coverages from at least two overlapping sub-areas, applying (1800) one or more predefined conflict resolution rules to determine a respective overlapping area cycle length coverage based on the conflicting sub-area cycle length coverages;
and indicating (1920) sub-areas and overlapping areas with respective cycle length coverages equal to or above the atrial flutter type threshold as areas including a potential micro re-entry.

10. The method of claim 9, wherein the sub-areas have a circular shape and the overlapping areas between two overlapping circles account for 1/5 to 1/3 of the sub-area diameter.

11. The method of claim 9, wherein the sub-areas have a circular shape with a diameter between 2 cm and 4 cm, and wherein the circle centers of two neighboring sub-areas have a distance between 0,5 cm and 1,5 cm.

12. The method of any of claims 9 to 11, wherein the one or more predefined conflict resolution rules are selected from the group of rules including: assigning the conflicting sub-area cycle length coverage with the highest value to the respective overlapping area cycle length coverage; assigning the average value of the conflicting sub-area cycle length coverages to the respective overlapping area cycle length coverage; and assigning a weighted average value of the conflicting sub-area cycle length coverages to the respective overlapping area cycle length coverage.

13. A computer program product for supporting diagnostic analysis of atrial flutter types, the computer program product when loaded into a memory of a computing device and executed by at least one processor of the computing device executes the steps of the computer-implemented method according to any one of the claims 7 to 12.

## Patentansprüche

1. Entscheidungsunterstützungscomputersystem (100) zum Unterstützen der diagnostischen Analyse von Vorhofflattertypen, Folgendes umfassend:
eine Schnittstellenkomponente (110), die konfiguriert ist, um ein elektrisches Referenzsignal (RS) zu empfangen, das durch einen Referenzsensor (CSCS) erzeugt wird, wobei das Referenzsignal (RS) eine Zeitreferenz für die elektrische Aktivität eines oder mehrerer Vorhöfe eines Patienten bereitstellt, wobei das Zeitintervall zwischen zwei aufeinanderfolgenden elektrischen Aktivitäten als Basiszykluslänge (*basic cycle length* - BCL) bezeichnet wird und ferner konfiguriert ist, um mehrere elektrischen Signale (P1 bis Pi) zu empfangen, die durch einen oder mehrere weiteren Sensoren (S1 bis Sn) erzeugt werden, wobei sich die mehreren elektrischen Signale (P1 bis Pi) auf mehrere Standorte in dem einen oder den mehreren Vorhöfen beziehen, wobei die mehreren Standorte Standorte umfassen, die sich von dem Standort des Referenzsensors (CSCS) unterscheiden;
eine Signalanalysatorkomponente (120), die konfiguriert ist, um für jedes Signal von wenigstens einer Teilmenge der empfangenen Signale, die von Standorten innerhalb eines ausgewählten Bereichs (500) des einen oder der mehreren Vorhöfe stammen, ein aktives Intervall (ai1 bis ai8) zu bestimmen, wenn eine elektrische Aktivität auftritt, und ein Ruheintervall, wenn keine elektrische Aktivität auftritt; und konfiguriert ist, um, basierend auf den bestimmten Intervallen, eine Teilmengenzykluslängenreichweite als das Verhältnis der Aktivitätsdauer (*duration of activity -* DoA) und der Basiszykluslänge (BCL) zu berechnen, wobei die Aktivitätsdauer die aktiven Intervalle für die vollständige Teilmenge von Signalen beinhaltet; und ferner konfiguriert ist, um einen Booleschen ODER-Operator auf die bestimmten Aktivitäts- und Ruheintervalle der jeweiligen Signale anzuwenden, so dass jedes Aktivitätsintervall eines Signals zu der Aktivitätsdauer zu der Aktivitätsdauer beiträgt, und keine Aktivität in den gesamten Signalen zu der Ruhedauer beiträgt; und
eine Visualisierungskomponente (130), die konfiguriert ist, um den ausgewählten Bereich als einen Bereich anzuzeigen, der eine mögliche Fokusquelle beinhaltet, wenn die Teilmengenzykluslängenreichweite kleiner als ein vordefiniertes Verhältnis ist, und andernfalls, um den ausgewählten Bereich als einen Bereich anzugeben, der einen möglichen Wiedereintritt beinhaltet.

2. System nach Anspruch 1, wobei der ausgewählte Bereich einem Atrium entspricht.

3. System nach einem der vorhergehenden Ansprüche, wobei das vordefinierte Verhältnis zwischen 75 % und 100 % liegt.

4. System nach einem der vorhergehenden Ansprüche, wobei bei Erfassung eines möglichen Wiedereintritts in den ausgewählten Bereich die Signalanalysatorkomponente ferner zu Folgendem konfiguriert ist:
Auswählen mehrerer Teilbereiche (sa 1 bis sa4) innerhalb des ursprünglich ausgewählten Bereichs (500), wobei die Teilbereiche eine Größe aufweisen, die kleiner als die Größe des ausgewählten Bereichs ist, und wobei jeder Teilbereich einen Überlappungsbereich (oa12 bis oa34) mit wenigstens einem weiteren Teilbereich aufweist;
Berechnen für jeden Teilbereich (sa 1 bis sa4) der jeweiligen Teilbereichszykluslängenreichweite;
für jeden Überlappungsbereich mit entgegengesetzten Teilbereichszykluslängenreichweiten aus wenigstens zwei Überlappungsteilbereichen eine oder mehrere vordefinierte Konfliktlösungsregeln anzuwenden, um eine jeweilige Überlappungsbereichszykluslängenreichweite, basierend auf den entgegengesetzten Teilbereichszykluslängenreichweiten, zu bestimmen; und
wobei die Visualisierungskomponente (130) ferner für Folgendes konfiguriert ist:
Angeben von Teilbereichen und Überlappungsbereichen mit jeweiligen Zykluslängenreichweiten, die gleich oder über dem Vorhofflattertypschwellenwert liegen, als Bereiche, die einen möglichen Mikro-Wiedereintritt beinhalten.

5. System nach Anspruch 4, wobei die Teilbereiche eine Kreisform aufweisen und die Überlappungsbereiche zwischen zwei Überlappungskreisen 1/5 bis 1/3 des Teilbereichsdurchmessers ausmachen, oder wobei die Teilbereiche eine Kreisform mit einem Durchmesser zwischen 2 cm und 4 cm aufweisen, wobei die Kreismittelpunkte zweier benachbarter Teilbereiche einen Abstand zwischen 0,5 cm und 1,5 cm aufweisen.

6. System nach Anspruch 4 oder 5, wobei die eine oder mehreren vordefinierten Konfliktlösungsregeln aus der Gruppe von Regeln ausgewählt sind, die Folgendes beinhalten: Zuweisen der entgegengesetzten Teilbereichszykluslängenreichweite mit dem höchsten Wert zu der jeweiligen Überlappungsbereichszykluslängenreichweite; Zuweisen des Durchschnittswerts der entgegengesetzten Teilbereichszykluslängenreichweiten zu der jeweiligen Überlappungsbereichszykluslängenreichweite; und Zuweisen eines gewichteten Mittelwerts der entgegengesetzten Teilbereichszykluslängenreichweiten zu der jeweiligen Überlappungsbereichszykluslängenreichweite.

7. Computerimplementiertes Verfahren (1000) zum Unterstützen der diagnostischen Analyse von Vorhofflattertypen, wobei das Verfahren Folgendes umfasst:
Empfangen (1100) eines elektrischen Referenzsignals, das durch ein Referenzsensor erzeugt wird, wobei das Referenzsignal eine Zeitreferenz für die elektrische Aktivität eines oder mehrerer Vorhöfe eines Patienten bereitstellt, wobei das Zeitintervall zwischen zwei aufeinanderfolgenden elektrischen Aktivitäten als Basiszykluslänge bezeichnet wird;
Empfangen (1200) mehrerer elektrischer Signale, die durch einen oder mehrere weitere Sensoren erzeugt werden, wobei sich die mehreren elektrischen Signale auf mehrere Standorte in dem einen oder den mehreren Vorhöfen beziehen, wobei die mehreren Standorte Standorte umfassen, die sich von dem Standort des Referenzsensors unterscheiden;
Bestimmen (1300), für jedes Signal von wenigstens einer Teilmenge der empfangenen Signale, die von Standorten innerhalb eines ausgewählten Bereichs des einen oder der mehreren Vorhöfe stammen, eines aktiven Intervalls, wenn elektrische Aktivität auftritt, und eines Ruheintervalls, wenn keine elektrische Aktivität auftritt;
basierend auf den bestimmten Intervallen, Berechnen (1400) einer Teilmengenzykluslängenreichweite als Verhältnis der Aktivitätsdauer (DoA) und der Basiszykluslänge (BCL), durch Anwenden eines Booleschen ODER-Operator auf die bestimmten Aktivitäts- und Ruheintervalle der jeweiligen Signale, so dass jedes Aktivitätsintervall eines Signals zu der Aktivitätsdauer beiträgt, und keine Aktivität in den gesamten Signalen zu der Ruhedauer beiträgt, wobei die Aktivitätsdauer (DoA) die aktiven Intervalle für die gesamte Teilmenge von Signalen beinhaltet;
falls die Teilmengenzykluslängenreichweite kleiner als ein vordefiniertes Verhältnis ist, dann Angeben (1510) des ausgewählten Bereichs als ein Bereich, der eine mögliche Fokalquelle beinhaltet, andernfalls Angeben (1520) des ausgewählten Bereichs als einen Bereich, der einen möglichen Wiedereintritt beinhaltet.

8. Verfahren nach einem der Ansprüche 7, wobei das vordefinierte definierte Verhältnis zwischen 75 % und 100 % liegt.

9. Verfahren nach einem der Ansprüche 7 bis 8, wobei wenn der ausgewählte Bereich einen möglichen Wiedereintritt beinhaltet, das Verfahren ferner Folgendes umfasst:
Auswählen (1600) mehrerer Teilbereiche innerhalb des ursprünglich ausgewählten Bereichs, wobei die Teilbereiche eine Größe aufweisen, die kleiner als die Größe des ausgewählten Bereichs ist, und wobei jeder Teilbereich einen Überlappungsbereich mit wenigstens einem weiteren Teilbereich aufweist;
Berechnen (1700) für jeden Teilbereich die jeweilige Teilbereichszykluslängenreichweite;
für jeden Überlappungsbereich mit entgegengesetzten Teilbereichszykluslängenreichweiteen aus wenigstens zwei Überlappungsteilbereichen, Anwenden (1800) einer oder mehrerer vordefinierter Konfliktlösungsregeln, um eine jeweilige Überlappungsbereichszykluslängenreichweite, basierend auf der entgegengesetzten Teilbereichszykluslängenreichweite, zu bestimmen;
und Angeben (1920) von Teilbereichen und Überlappungsbereichen mit jeweiligen Zykluslängenreichweiten, die gleich oder über der Schwelle des Vorhofflattertyps liegen, als Bereiche, die einen möglichen Mikro-Wiedereintritt beinhalten.

10. Verfahren nach Anspruch 9, wobei die Teilbereiche eine Kreisform aufweisen und die Überlappungsbereiche zwischen zwei Überlappungskreisen 1/5 bis 1/3 des Teilbereichsdurchmessers ausmachen.

11. Verfahren nach Anspruch 9, wobei die Teilbereiche eine Kreisform mit einem Durchmesser zwischen 2 cm und 4 cm aufweisen, und wobei die Kreismittelpunkte zweier benachbarter Teilbereiche einen Abstand zwischen 0,5 cm und 1,5 cm aufweisen.

12. Verfahren nach einem der Ansprüche 9 bis 11, wobei die eine oder mehreren vordefinierten Konfliktlösungsregeln aus der Gruppe von Regeln ausgewählt sind, die Folgendes beinhalten: Zuweisen der entgegengesetzten Teilbereichszykluslängenreichweite mit dem höchsten Wert zu der jeweiligen Überlappungsbereichszykluslängenreichweite; Zuweisen des Durchschnittswerts der entgegengesetzten Teilbereichszykluslängenreichweiten zu der jeweiligen Überlappungsbereichszykluslängenreichweite; und Zuweisen eines gewichteten Mittelwerts der entgegengesetzten Teilbereichszykluslängenreichweiten zu der jeweiligen Überlappungsbereichszykluslängenreichweite.

13. Computerprogrammprodukt zum Unterstützen der diagnostischen Analyse von Vorhofflattertypen, wobei das Computerprogrammprodukt, wenn es in einen Speicher einer Rechenvorrichtung geladen und durch wenigstens einen Prozessor der Rechenvorrichtung ausgeführt wird, die Schritte des computerimplementierten Verfahrens nach einem der Ansprüche 7 bis 12 ausführt.

## Revendications

1. Système informatique d'aide à la décision (100) destiné à prendre en charge l'analyse diagnostique de types de flutter auriculaire, comprenant :
un composant d'interface (110) configuré pour recevoir un signal de référence électrique (RS) généré par un capteur de référence (CSCS), le signal de référence (RS) fournissant une référence temporelle pour l'activité électrique d'une ou plusieurs oreillettes d'un patient, l'intervalle de temps entre deux activités électriques ultérieures étant appelé longueur de cycle de base (BCL) et en outre configurée pour recevoir une pluralité de signaux électriques (P1 à Pi) générés par un ou plusieurs autres capteurs (S1 à Sn), la pluralité de signaux électriques (P1 à Pi) concernant une pluralité d'emplacements dans lesdites oreillettes, la pluralité d'emplacements comprenant des emplacements différents de l'emplacement du capteur de référence (CSCS) ;
un composant analyseur de signal (120) configuré pour déterminer, pour chaque signal d'au moins un sous-ensemble des signaux reçus provenant d'emplacements dans une zone sélectionnée (500) desdites oreillettes, un intervalle actif (ai1 à ai8) lorsqu'une activité électrique se produit et un intervalle de repos lorsqu'aucune activité électrique ne se produit ; et
configuré pour calculer, sur la base des intervalles déterminés, une couverture de longueur de cycle de sous-ensemble en tant que rapport de la durée d'activité (DoA) et de la longueur de cycle de base (BCL), la durée d'activité comportant les intervalles actifs pour l'ensemble du sous-ensemble de signaux ; et
configuré en outre pour appliquer un booléen OU un opérateur à l'activité déterminée et aux intervalles de repos des signaux respectifs de sorte que tout intervalle d'activité de tout signal contribue à la durée d'activité, et qu'aucune activité dans tous les signaux ne contribue à la durée du repos ; et
un composant visualisateur (130) configuré pour indiquer la zone sélectionnée en tant que zone comportant une source focale potentielle si la couverture de longueur de cycle de sous-ensemble est inférieure à un rapport prédéfini, et sinon pour indiquer la zone sélectionnée en tant que zone comportant une nouvelle entrée potentielle.

2. Système selon la revendication 1, dans lequel la zone sélectionnée correspond à une oreillette.

3. Système selon l'une quelconque des revendications précédentes, dans lequel le rapport défini prédéfini est compris entre 75 % et 100 %.

4. Système selon l'une quelconque des revendications précédentes, dans lequel, lors de la détection d'une nouvelle entrée potentielle dans la zone sélectionnée, le composant analyseur de signal est en outre configuré pour :
sélectionner une pluralité de sous-zones (sa1 à sa4) à l'intérieur de la zone sélectionnée à l'origine (500), les sous-zones étant d'une taille qui est plus petite que la taille de la zone sélectionnée, et chaque sous-zone ayant une zone de chevauchement (oa12 à oa34) avec au moins une autre sous-zone ;
calculer pour chaque sous-zone (sa1 à sa 4) la couverture de longueur de cycle de sous-zone respective ;
pour chaque zone de chevauchement avec des couvertures de longueur de cycle de sous-zone en conflit d'au moins deux sous-zones de chevauchement,
appliquer une ou plusieurs règles de résolution de conflit prédéfinies pour déterminer une couverture de longueur de cycle de zone de chevauchement respective sur la base des couvertures de longueur de cycle de sous-zone en conflit ; et
le composant visualisateur (130) est en outre configuré pour :
indiquer des sous-zones et des zones de chevauchement avec des couvertures de longueur de cycle respectives égales ou supérieures au seuil de type de flutter auriculaire en tant que zones comportant une nouvelle entrée micro potentielle.

5. Système selon la revendication 4, dans lequel les sous-zones ont une forme circulaire et les zones de chevauchement entre deux cercles de chevauchement représentent 1/5 à 1/3 du diamètre de la sous-zone, ou les sous-zones ayant une forme circulaire avec un diamètre compris entre 2 cm et 4 cm, et les centres des cercles de deux sous-zones voisines ayant une distance comprise entre 0,5 cm et 1,5 cm.

6. Système selon la revendication 4 ou 5, dans lequel lesdites règles de résolution de conflit prédéfinies sont sélectionnées dans le groupe de règles comportant : l'attribution de la couverture de longueur de cycle de sous-zone en conflit avec la valeur la plus élevée à la couverture de longueur de cycle de zone de chevauchement respective ; l'attribution de la valeur moyenne des couvertures de longueur de cycle de sous-zone en conflit à la couverture de longueur de cycle de zone de chevauchement respective ; et l'attribution d'une valeur moyenne pondérée des couvertures de longueur de cycle de sous-zone en conflit à la couverture de longueur de cycle de zone de chevauchement respective.

7. Procédé (1000) mis en oeuvre par ordinateur destiné à prendre en charge l'analyse diagnostique des types de flutter auriculaire, le procédé comprenant :
la réception (1100) d'un signal de référence électrique généré par un capteur de référence, le signal de référence fournissant une référence temporelle pour l'activité électrique d'une ou plusieurs oreillettes d'un patient, l'intervalle de temps entre deux activités électriques ultérieures étant appelé longueur de cycle de base ;
la réception (1200) d'une pluralité de signaux électriques générés par un ou plusieurs autres capteurs, la pluralité de signaux électriques se rapportant à une pluralité d'emplacements dans lesdites oreillettes, la pluralité d'emplacements comprenant des emplacements différents de l'emplacement du capteur de référence ;
la détermination (1300), pour chaque signal d'au moins un sous-ensemble des signaux reçus provenant d'emplacements dans une zone sélectionnée desdites oreillettes, d'un intervalle actif lorsqu'une activité électrique se produit et d'un intervalle de repos lorsqu'aucune activité électrique ne se produit ;
sur la base des intervalles déterminés, le calcul (1400) d'une couverture de longueur de cycle de sous-ensemble en tant que rapport de la durée d'activité (DoA) et de la longueur de cycle de base (BCL) en appliquant un booléen OU un opérateur à l'activité déterminée et aux intervalles de repos des signaux respectifs de sorte que tout intervalle d'activité de tout signal contribue à la durée de l'activité et qu'aucune activité de tous les signaux ne contribue à la durée de repos, la durée d'activité (DoA) comportant les intervalles actifs pour l'ensemble du sous-ensemble de signaux ;
si la couverture de longueur de cycle de sous-ensemble est inférieure à un rapport prédéfini, alors l'indication (1510) de la zone sélectionnée en tant que zone comportant une source focale potentielle, sinon l'indication (1520) de la zone sélectionnée en tant que zone comportant une nouvelle entrée potentielle.

8. Procédé selon l'une quelconque des revendications 7, dans lequel le rapport défini prédéfini est compris entre 75 % et 100 %.

9. Procédé selon l'une quelconque des revendications 7 à 8, si la zone sélectionnée comporte une nouvelle entrée potentielle, le procédé comprenant en outre :
la sélection (1600) d'une pluralité de sous-zones dans la zone sélectionnée à l'origine, les sous-zones ayant une taille qui est plus petite que la taille de la zone sélectionnée, et chaque sous-zone ayant une zone de chevauchement avec au moins une autre sous-zone ;
le calcul (1700) pour chaque sous-zone de la couverture de longueur de cycle de sous-zone respective ;
pour chaque zone de chevauchement avec des couvertures de longueur de cycle de sous-zone en conflit provenant d'au moins deux sous-zones de chevauchement, l'application (1800) d'une ou plusieurs règles de résolution de conflit prédéfinies pour déterminer une couverture de longueur de cycle de zone de chevauchement respective sur la base des couvertures de longueur de cycle de sous-zone en conflit ;
et l'indication (1920) de sous-zones et de zones de chevauchement avec des couvertures de longueur de cycle respectives égales ou supérieures au seuil de type de flutter auriculaire en tant que zones comportant une nouvelle entrée micro potentielle.

10. Procédé selon la revendication 9, dans lequel les sous-zones ont une forme circulaire et les zones de chevauchement entre deux cercles de chevauchement représentent 1/5 à 1/3 du diamètre de la sous-zone.

11. Procédé selon la revendication 9, dans lequel les sous-zones ont une forme circulaire avec un diamètre entre 2 cm et 4 cm, et les centres des cercles de deux sous-zones voisines ayant une distance entre 0,5 cm et 1,5 cm.

12. Procédé selon l'une quelconque des revendications 9 à 11, dans lequel lesdites règles de résolution de conflit prédéfinies sont sélectionnées dans le groupe de règles comportant : l'attribution de la couverture de longueur de cycle de sous-zone en conflit avec la valeur la plus élevée à la couverture de longueur de cycle de zone de chevauchement respective ; l'attribution de la valeur moyenne des couvertures de longueur de cycle de sous-zone en conflit à la couverture de longueur de cycle de zone de chevauchement respective ; et l'attribution d'une valeur moyenne pondérée des couvertures de longueur de cycle de sous-zone en conflit à la couverture de longueur de cycle de zone de chevauchement respective.

13. Produit de programme informatique destiné à prendre en charge l'analyse diagnostique des types de flutter auriculaire, le produit de programme informatique, lorsqu'il est chargé dans une mémoire d'un dispositif informatique et exécuté par au moins un processeur du dispositif informatique, exécute les étapes du procédé mis en oeuvre par ordinateur selon l'une quelconque des revendications 7 à 12.
